(19) **Europäisches Patentamt / European Patent Office / Office européen des brevets**

(11) **EP 4 189 684 B1**

(12) **EUROPEAN PATENT SPECIFICATION**

(45) Date of publication and mention
of the grant of the patent:
**10.09.2025 Bulletin 2025/37**

(21) Application number: **21715150.5**

(22) Date of filing: **12.03.2021**

(51) International Patent Classification (IPC):
**G16B 15/30** (2019.01)    **G16B 40/20** (2019.01)

(52) Cooperative Patent Classification (CPC):
**G16B 15/30; G16B 40/20**

(86) International application number:
**PCT/EP2021/056387**

(87) International publication number:
**WO 2022/078633 (21.04.2022 Gazette 2022/16)**

(54) **MULTIPLE INSTANCE LEARNING FOR PEPTIDE-MHC PRESENTATION PREDICTION**

LERNEN MEHRERER INSTANZEN ZUR VORHERSAGE DER PRÄSENTATION VON PEPTID-MHC

APPRENTISSAGE À INSTANCES MULTIPLES POUR PRÉDICTION DE PRÉSENTATION PEPTIDE-MHC

---

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.10.2020 EP 20201557**

(43) Date of publication of application:
**07.06.2023 Bulletin 2023/23**

(73) Proprietor: **NEC Corporation**
**Minato-ku**
**Tokyo 108-8001 (JP)**

(72) Inventors:
• **CHENG, Jun**
**69115 Heidelberg (DE)**
• **MALONE, Brandon**
**69221 Dossenheim (DE)**

(74) Representative: **Ullrich & Naumann PartG mbB**
**Schneidmühlstrasse 21**
**69115 Heidelberg (DE)**

(56) References cited:
**US-A1- 2015 278 441**

• **YASSER EL-MANZALAWY ET AL**: "Predicting MHC-II Binding Affinity Using Multiple Instance Regression", IEEE/ACM TRANSACTIONS ON COMPUTATIONAL BIOLOGY AND BIOINFORMATICS, IEEE SERVICE CENTER, NEW YORK, NY, US, vol. 8, no. 4, 23 September 2010 (2010-09-23), pages 1067 - 1079, XP058004750, ISSN: 1545-5963, DOI: 10.1109/TCBB.2010.94
• **NICO PFEIFER ET AL**: "Multiple Instance Learning Allows MHC Class II Epitope Predictions Across Alleles", 15 September 2008, ALGORITHMS IN BIOINFORMATICS; [LECTURE NOTES IN COMPUTER SCIENCE], SPRINGER BERLIN HEIDELBERG, BERLIN, HEIDELBERG, PAGE(S) 210 - 221, ISBN: 978-3-540-87360-0, XP019105333
• **YICHANG XU ET AL**: "MHC2MIL: a novel multiple instance learning based method for MHC-II peptide binding prediction by considering peptide flanking region and residue positions", BMC GENOMICS, BIOMED CENTRAL, vol. 15, no. Suppl 9, 8 December 2014 (2014-12-08), pages S9, XP021204646, ISSN: 1471-2164, DOI: 10.1186/1471-2164-15-S9-S9
• **ANONYMOUS**: "Multiple instance learning - Wikipedia", 14 August 2020 (2020-08-14), XP055812637, Retrieved from the Internet <URL:https://en.wikipedia.org/w/index.php?title=Multiple_instance_learning&oldid=972908596> [retrieved on 20210610]

---

**(Cont. next page)**

- PARK SEONGOH ET AL: "Bayesian multiple instance regression for modeling immunogenic neoantigens", STATISTICAL METHODS IN MEDICAL RESEARCH, vol. 29, no. 10, 13 May 2020 (2020-05-13), US, pages 3032 - 3047, XP055839545, ISSN: 0962-2802, Retrieved from the Internet <URL:https://journals.sagepub.com/doi/pdf/10.1177/0962280220914321> DOI: 10.1177/0962280220914321
- KANDEMIR MELIH ET AL: "Digital pathology: Multiple instance learning can detect Barrett's cancer", 2014 IEEE 11TH INTERNATIONAL SYMPOSIUM ON BIOMEDICAL IMAGING (ISBI), IEEE, 29 April 2014 (2014-04-29), pages 1348 - 1351, XP032779052, DOI: 10.1109/ISBI.2014.6868127
- STEFANOS ANGELIDIS ET AL: "Multiple Instance Learning Networks for Fine-Grained Sentiment Analysis", ARXIV.ORG, CORNELL UNIVERSITY LIBRARY, 201 OLIN LIBRARY CORNELL UNIVERSITY ITHACA, NY 14853, 27 November 2017 (2017-11-27), XP081308314

**Description**

The present invention relates to a computer-implemented method and system for predicting binding and presentation of peptides by MHC molecules.

Furthermore, the present invention relates to a computer-implemented method for performing multiple instance learning, MIL.

The adaptive immune system plays a central role in immune response against foreign molecules, such as pathogens or cancerous cells. The adaptive immune system has two major branches: humoral immunity, which concerns antibody generation, and cell-mediated immunity, which entails stimulation of cytotoxic CD8+ T cells among other things.

The major histocompatibility complex (MHC) class II plays an important role in both humoral and cell-mediated immunity (for reference, see Murphy, K. and Weaver, C., 2016. Janeway's immunobiology. Garland science). The primary role of MHC class II is to bind to and then present peptide sequences, which are short amino acid sequences, from exogenous proteins on the cell surface. This peptide-MHC complex leads to the stimulation of CD4+ T cells, or "helper T cells". The helper T cells may then stimulate either the humoral or cell-mediated immune response pathways.

MHC class II molecules are mostly found in "professional" antigen presenting cells, such as dendritic cells. Among the MHC class II molecules, each person typically has two alleles each from the HLA-DQ and HLA-DP gene families, while they may have up to 10 alleles from the HLA-DR gene family (for reference, see Choo, S.Y., 2007. The HLA system: genetics, immunology, clinical testing, and clinical implications. Yonsei medical journal, 48(1), pp.11-23). Importantly, different people have different MHC alleles, although some alleles are more common than others. The different versions of the MHC alleles have different amino acid sequences and structures, and these differences affect to which peptides the MHC alleles bind and present on the cell surface.

The presentation of peptides to T cells involves a series of processes. Important steps include binding between MHC molecules and peptides, as well as presentation of the peptide-MHC complex to the cell surface. Mass spectrometry can be used to detect peptides eluted from the cell surface to determine peptide presentation (for reference, see Purcell, A.W., Ramarathinam, S.H. and Ternette, N., 2019. Mass spectrometry-based identification of MHC-bound peptides for immunopeptidomics. Nature protocols, 14(6), p.1687). Thousands of data points have been generated by such assays for hundreds of different MHC molecules (for reference, see Vita, R., Mahajan, S., Overton, J.A., Dhanda, S.K., Martini, S., Cantrell, J.R., Wheeler, D.K., Sette, A. and Peters, B., 2019. The immune epitope database (IEDB): 2018 update. Nucleic acids research, 47(D1), pp.D339-D343). As mentioned, each person has multiple MHC class II molecules; thus, typical mass spectrometry experiments cannot precisely identify the MHC molecule which presented a particular peptide. Another limitation of mass spectrometry is that it can only indicate peptides which were detected; that is, it cannot generate "negative" data points. It is therefore an important challenge to use this experimental data in order to train machine learning models to predict peptide-MHC presentation.

The document Yasser El-Manzalawy et al.: "Predicting MHC-II Binding Affinity Using Multiple Instance Regression", in IEEE/ACM Transactions on Computational Biology and Bioinformatics, Vol. 8, No. 4, p. 1067-1079, July/August 2011 focuses on the problems of qualitatively and quantitatively predicting flexible length MHC-II peptides and discloses an approach that formulates the problems as multiple instance learning and multiple instance regression problems, respectively. Based on this formulation, the document describes a method for predicting MHC-II binding affinity using multiple instance regression.

The document Nico Pfeifer and Oliver Kohlbacher: "Multiple Instance Learning Allows MHC Class II Epitope Predictions Across Alleles", in Algorithms in Computer Science, Springer-Verlag Berlin Heidelberg, p. 210-221, 15. September 2008 discloses an approach to transform the problem of MHC class II binding peptide prediction into a machine learning problem, namely multiple instance learning. In view of the scarcity of data, the document discloses a method for training a classifier of an allele that, instead of using binding allele data of the target allele, uses binding peptide data from other alleles and similarities between the structures of the MHC class II alleles to guide the learning process.

The document Yichang Xu et al.: "MHC2MIL: a novel multiple instance learning based method for MHC-II peptide binding prediction by considering peptide flanking region and residue positions", in BMC Genomics, vol. 15, no. Suppl 9, 8 December 2014 discloses a multiple instance learning based method to predict MHC-II binding peptides. Each peptide is deemed as a bag, and some substrings of the peptide as the instances in the bag. The method is able to deal with instances of both lengths of 9 (9 amino acids) and 11 (11 amino acids), simultaneously.

US 2015/0278441 A1 discloses a method for peptide binding prediction, comprising: receiving a peptide sequence descriptor and contacting amino acid descriptors on MHC protein-peptide interaction structure; generating a model with one or an ensemble of high-order neural network explicit high-order interactions of feature descriptors of both peptides and MHC class proteins; pre-training the model by high-order semi-Restricted Boltzmann machine (RBM) or high-order denoising autoencoder; integrating both peptide sequence information and structural information of MHC protein-peptide interaction complexes; applying the deep learning model for T-cell epitope prediction; and generating a prediction as a binary output or continuous output with initial model parameters pre-trained using available binary output data. The model can be trained on peptides of a fixed length. On the other hand, for MHC II proteins with input peptides that vary in length, the document proposes using sliding window or amino acid skipping to get a bag of peptides of a desired fixed length, and using output score averaging/maximization or multiple instance learning to train high-order neural networks for peptide binding prediction.

It is an object of the present invention to improve and further develop methods and systems of the initially described type in such a way that the prediction performance is improved.

In accordance with the invention, the aforementioned object is accomplished by a computer-implemented method for predicting binding and presentation of peptides by MHC molecules, the method comprising: collecting or generating training data, wherein the training data includes a set of MHC molecules present in a biological sample as well as a set of observed peptide sequences that are presented by at least one of the MHC molecules present in the biological sample, wherein it is not known to which specific of the MHC molecules a peptide sequence is bound, wherein the training data is provided in form of a set of triples $\{s_i, A_i, y_i\}$, where $s_i$ is a peptide sequence, $A_i = \{a_1, \cdots, a_m\}$ is a set of m MHC molecules associated with a biological sample, and $y_i$ is a binary label indicating whether $s_i$ was found to be presented by any of the MHC molecules in $A_i$, and wherein the training data are organized in bags with each bag having a set of training instances, wherein labels are known for the bags, but unknown for the training instances; using a loss function to train an MIL classifier $f_\theta$ at an instance-level, wherein the MIL classifier $f_\theta$ is trained to predict whether a particular peptide sequence will be presented by any of the MHC molecules present in the biological sample; wherein the parameters of the MIL classifier $f_\theta$ are trained by a loss function $L(\theta)$ that includes a probability calibration function $\mathcal{C}$ to calibrate model confidence, the probability calibration function $\mathcal{C}$ being configured to predict in each training epoch $k + 1$ the probabilities $\hat{p}_i$ of $\hat{y}_i$ of the previous training epoch $k$, wherein $\hat{y}_i = \underset{j}{max}(f_\theta(x_{ij}))$ and $x_{ij}$ corresponds to the tuple $(s_i, a_j)$, where $s_i$ is the peptide and $a_j$ is the $j_{th}$ MHC molecule in $A_i$, wherein only individual training instances from positively labeled bags are weighted by the probability calibration function $\mathcal{C}$; and predicting the label of new instances by applying the MIL classifier $f_\theta$ directly and/or predicting the label of new bags by applying the MIL classifier $f_\theta$ to each instance of a respective bag and aggregating the results among all instances of the respective bag.

In further embodiments, a system for predicting binding and presentation of peptides by MHC molecules comprises one or more processors which, alone or in combination, are configured to allow for execution of any of the methods according to embodiments of the present invention.

In even further embodiments, a tangible, non-transitory computer-readable medium comprises instructions which, upon execution on one or more processors cause the one or more processors, alone or in combination, to allow for execution of any of the methods according to embodiments of the present invention.

Embodiments of the invention provide an MIL algorithm, with application to peptide-MHC predictions with multiple MHC alleles. Embodiments of the invention allow efficient usage of typical peptide-MHC mass spectrometry data with multiple potential allele labels. The present disclosure focuses on predicting precisely binding and presentation of peptides by MHC alleles, which is an important step towards personalized T-cell-based vaccine design and immunotherapy.

In an embodiment, the present invention provides a computer-implemented method for performing multiple instance learning, the method comprising a first step of collecting or generating training data where the labels are only known for bags of instances. The method may further include training a classifier at an instance-level where individual training instances from the positively labeled bags are weighted by a calibrated current model confidence in the loss function with the training data from the first step. Based on the trained MIL classifier the method may then include predicting the label of new instances by applying the instance-level classifier directly, or predicting the label of new bags by applying the instance-level classifier to each instance and aggregating the scores among all instances within the bags.

According to the invention, the MIL classifier is trained by using a loss function that explicitly accounts for model confidence in the model predictions during training. The probabilities are calibrated by means of a probability calibration function to accurately reflect the current model confidence. In this context, training instances in the positively labeled bags are weighted by a calibrated current model confidence level.

There are several ways how to design and further develop the teaching of the present invention in an advantageous way. To this end it is to be referred to the dependent claims on the one hand and to the following explanation of preferred embodiments of the invention by way of example, illustrated by the figure on the other hand. In connection with the explanation of the preferred embodiments of the invention by the aid of the figure, generally preferred embodiments and further developments of the teaching will be explained. In the drawing

Fig. 1     is a schematic view illustrating a prediction scheme based on experimentally obtained data in accordance with an embodiment of the invention,

Fig. 2     is a schematic view illustrating bag label predictions by using a classifier predicting instance labels and by applying a pooling operation in accordance with an embodiment of the invention,

Fig. 3    is a schematic view illustrating a probability calibration function used to calibrate model confidence in accordance with an embodiment of the invention,

Fig. 4    is a schematic view illustrating a loss function modified to approximate negative samples with negative sampling in accordance with an embodiment of the invention,

Fig. 5    is a schematic view illustrating a personalized cancer vaccine design in accordance with an embodiment of the invention.

Predicting the binding and presentation between MHC molecules and peptides is an important step towards T-cell-based vaccine design and immunotherapy. Given the importance of the problem and the availability of the data, many methods have been developed to predict MHC-peptide binding and peptide presentation. In some approaches, a single model is trained specifically for each MHC allele; other approaches instead train a single model covering all MHC alleles (pan model). The prediction performances of MHC class I models have reached a high level (auROC > 0.98, for reference see Peters, M. E., Neumann, M., Iyyer, M., Gardner, M., Clark, C., Lee, K., and Zettlemoyer, L. (2018). Deep contextualized word representations. Proceedings of the 16th Annual Conference of the North American Chapter of the Association for Computational Linguistics: Human Language Technologies, 1, 2227-2237). On the other hand, models for class II still have limited performance. Despite recent progresses, there is still a need for better performing models. One significant limiting factor for MHC class II models is the limited amount of training data compared to class I. Thus, models that can efficiently use the limited available data and by transferring knowledge from other sources are extremely valuable.

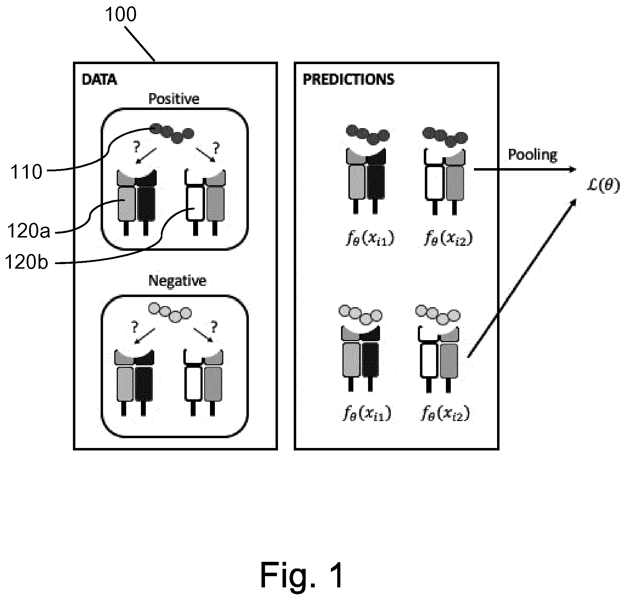

As already mentioned, predicting which peptide can or cannot be presented by which MHC molecule is crucial for neoantigen discovery and T-cell-based vaccines design, among other health-related problems. One important source of training data for such models is mass spectrometry. This technique identifies short peptides which are presented to the cell surface, due the MHC molecule(s) available in the cells. As indicated in the left part of Fig. 1, many mass spectrometry data 100 are generated with more than one MHC molecule in the cell, which means for a positive peptide 110 discovered with mass spectrometry, one or more MHC molecules 120a, 120b could be responsible for the presenting the peptide 110.

Embodiments of the present invention provide a method and a system which prioritize peptides for inclusion in a vaccine based on their likelihood to be presented on the cell surface by MHC molecules for a particular individual. In an embodiment, the prioritization is posed as a prediction problem, and a multiple instance learning (MIL) formulation is adopted to solve it. While prior work has also formulated this as an MIL problem, embodiments of the invention explicitly account for and calibrate model confidence during the learning process using a novel learning algorithm.

In standard supervised learning, labels are provided for each input sample. In some contexts, though, labels are instead assigned to *sets* or *bags* of inputs. In this setting, a bag of inputs is labeled as positive if it contains at least one positive input, otherwise the bag is labeled as negative.

As such, in accordance with an embodiment of the invention, the training data for multiple instance learning, MIL, may be defined as $X = \{x_1, x_2, ..., x_N\}$ and the associated bag labels as $\{y_1, y_2, ..., y_N\}$. Each bag may have a set of instances, i.e., $X_i = \{x_{i1}, x_{i2}, ..., x_{im}\}$. MIL assumes each instance in the bag has a label $y_{ij} \in \{0,1\}$, but remains unknown in the training. Only labels $y_i$ for the bag are provided, namely as follows:

$$y_i = \begin{cases} 1 & \text{if } \exists\, j\; s.t.\; y_{ij} = 1 \\ 0 & \text{Otherwise} \end{cases}$$

An MIL classifier $f_\theta$ can either learn to predict the label of a new bag $f_\theta(X)$ (bag-level approach) or to predict the label of an instance $f_\theta(x_{ij})$ (instance-level approach). Embodiments of the invention focus on training classifiers predicting the label of instances, i.e. on the instance-level approach.

A classifier predicting the label of instances can be used to predict the label of a bag by applying a pooling operation $h(\cdot)$ on the predictions for all instances in the bag:

$$f_\theta(X) = h\big(f_\theta(x_{i1}), f_\theta(x_{i2}), ..., f_\theta(x_{im})\big),$$

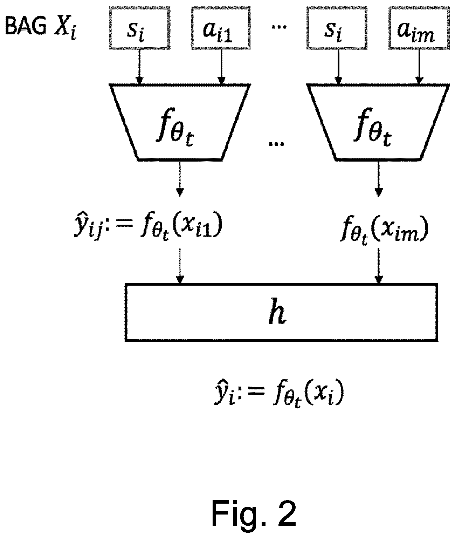

as indicated in the right part of Fig. 1 as well as in Fig. 2, where $s_i$ denote peptide sequences, $A_i = \{a_1, \cdots, a_m\}$ is a set of $m$ MHC molecules associated with a biological sample, and $y_i$ is a binary label indicating whether $s_i$ was found to be presented by any of the MHC molecules in $A_i$.

From the definition of the problem, it is required that $h(\cdot)$ is a permutation-invariant function, which means input order to the function has no influence on the result. The classifier $f_\theta$ may be trained using a loss function with the following form:

$$L(\theta) = \frac{1}{N}\sum_{i=1}^{N} Loss_i(y_i, h(f_\theta(x_{i1}), f_\theta(x_{i2}), \ldots, f_\theta(x_{im}))),$$

where $N$ is the number of bags and $M$ is the number of instances in each bag. Here, it should be noted that, in general, it is not required that all bags have the same number of instances.

According to some embodiments, the present invention provides methods and systems that include a multiple instance learning (MIL) approach based on the peptide-MHC presentation problem discussed above. The method may be performed in two phases, an offline training phase and an online prediction phase.

In the offline training phase, a prediction model will be trained which explicitly accounts for and calibrates model confidence, which is in contrast to prior work. The trained model is then used during the online prediction phase.

According to some embodiments, the present invention provides methods and systems for predicting binding and presentation of peptides by MHC molecules that are configured to receive, as input in the offline training phase, a set of observed peptides which are presented by at least one MHC molecule which was present in a biological sample, as well as the set of MHC molecules which are present in that sample. As already explained above, it is not known, however, to which specific MHC molecule a peptide was bound. This is exactly the kind of data produced by mass spectrometry experiments.

In an embodiment, a standard approach may be used to generate negative examples for training. It should be noted, however, that the applicability of the approach proposed in accordance with the present invention does not depend on how negative examples are created.

More specifically, the input may be provided in the form of a set of triples $\{s_i, A_i, y_i\}$, where $s_i$ is a peptide sequence, $A_i = \{a_1, \cdots, a_m\}$ is a set of $m$ MHC molecules associated with a biological sample, and $y_i$ is a binary label indicating whether $s_i$ was found to be presented by any of the MHC molecules in $A_i$.

The goal of the offline training phase is to train a machine learning model $f_\theta$ which takes as input $X_i = (s_i, A_i)$ and correctly predicts $y_i$. One example of $f_\theta$ is a pretrained bidirectional encoder representations from transformers (BERT) model. However, as will be appreciated by those skilled in the art, other model types are likewise possible. The only restriction is that the model must provide a probability $p(y_{ij} = 1|s_i, a_j)$ associated with the prediction for each instance.

According to an embodiment of the invention, each peptide is associated with a bag of alleles. The bag is labeled as positive if at least one of the allele presented the peptide, otherwise the bag is labelled as negative. The training data may be modelled as a multiple instance learning (MIL) problem. Here, the ith bag with m alleles is denoted as $A_i = \{a_{i1}, a_{i2}, \ldots, a_{im}\}$ and the corresponding peptide sequence as $s_i$. At each training step, the probability $p(y_{ij} = 1|x_{ij})$ of every instance $(a_{ij}, s_i)$ in the bag may be predicted as $\hat{y}_{ij} = f_\theta(a_{ij}, s_i)$ with the neural network model $f_\theta$. A symmetric pooling operator may be used to pool the prediction of the bag from the predictions of instances within it. To incorporate the uncertainty of the deconvolution operation, at each training epoch each positive data point i from deconvolution may be weighted by a calibrated predicted probability of being positive $\mathcal{C}(\hat{p}_i)$.

According to an embodiment of the invention, the parameters of the model may then be learned according to the following loss function:

$$\mathcal{L}(\theta) = -\frac{1}{N_{Pos}}\sum_{i\in Pos}^{N_{Pos}}(\mathcal{C}(\hat{p}_i) \cdot w \cdot \log(\hat{y}_i)) - \frac{1}{N_{Neg}}\sum_{i\in Neg}^{N_{Neg}}\frac{1}{m_i}\sum_{j=1}^{m_i}\log\left(1 - \hat{y}_{ij}\right),$$

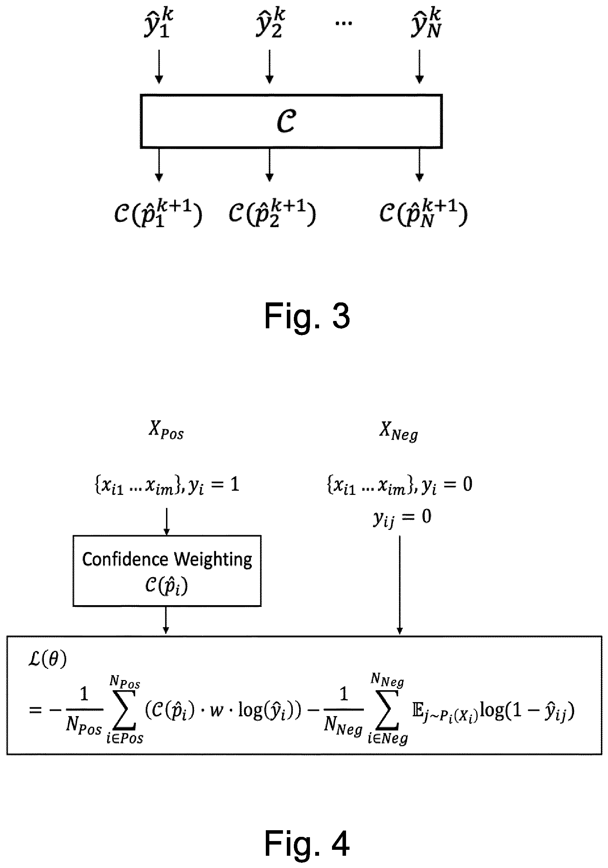

where $\hat{y}_i = \max_j(f_\theta(x_{ij}))$, $\hat{p}_i$ is the predicted probability of $\hat{y}_i$ of the previous training epoch of the model, $\mathcal{C}$ is a probability calibration function (FIG. 3), w is the weight for the positive class to count for class imbalance, and $x_{ij}$ corresponds to the tuple $(s_i, a_j)$, where $s_i$ is the peptide and $a_j$ is the $j_{th}$ MHC molecule in $A_i$. According to the embodiment illustrated in Fig. 3, the probability calibration function $\mathcal{C}$ may be configured to receive as input the values $\hat{y}_i$ of a current training epoch $k$ of the model and may calculate calibrated probabilities $\hat{p}_i$ for a subsequent training epoch $k+1$ of the model. With respect to the instance weighting it should be noted that only the instances in positively labeled bags are weighted with calibrated model confidences, in accordance with embodiments of the invention, while negative samples are not weighted (since there is no uncertainty with the labels of negative classes). In this context it may be provided that either all negative samples are used or that negative sampling is performed if negative bags are large and computation is limited.

The given formulation incorporates all negative instances in all of the negative bags. However, in cases for which there are many negative bags, this is computationally challenging. Therefore, according to an alternative embodiment, it may be provided to approximate the negative samples with negative sampling, as shown in Fig. 4. Accordingly, the above loss function may be modified as:

$$\mathcal{L}(\theta) = -\frac{1}{N_{Pos}} \sum_{i \in Pos}^{N_{Pos}} (\mathcal{C}(\hat{p}_i) \cdot w \cdot \log(\hat{y}_i)) - \frac{1}{N_{Neg}} \sum_{i \in Neg}^{N_{Neg}} \mathbb{E}_{j \sim P_i(X_i)} \log(1 - \hat{y}_{ij})$$

For computational reason, negative sampling may be performed with a probability distribution $P_i(X_i)$ instead of using all negative samples. According to an embodiment, for the MHC-peptide presentation problem, one may choose to use the following delta distribution for $P_i(X_i) = P_i(x_{i1}, x_{i2}, ..., x_{im})$:

$$P_i(x_{ij}) = \begin{cases} 1 \text{ if } f_\theta(x_{ij}) = \max(f_\theta(x_{i1}), f_\theta(x_{i2}), ..., f_\theta(x_{im})) \\ 0 \qquad \text{otherwise} \end{cases}$$

That is, the method uses the most likely positive example predicted by the current model from the negative bag.

Considering the above, a multiple instance learning (MIL) algorithm according to an embodiment of the invention, with application to peptide-MHC predictions with multiple MHC alleles, can be stated as follows:

---

### Algorithm: Probability Reweighted Multiple Instance Learning

**Input**: Training data $\{X_i, y_i\}_{i \in 1...N}$, where $X_i := \{s_i, A_i\}, y_i \in \{0,1\}$;

Random initalize $\theta_0$ or transfer $\theta_0$ from a related task, $\theta_k \leftarrow \theta_0$, choose $w$

**while** not converge **do**:

    **for** $k$ in $0...N_{EPOCH}$:

    Predict bag labels with the current model $\hat{P} := \left\{ h\left( f_{\theta_k}(x_{ij}), ..., f_{\theta_k}(x_{im}) \right) \right\}_{i \in 1...N}$

    Train a probability calibration model $\mathcal{C}_k$ with $\{y_i, \text{logit}(\hat{p}_i)\}_{i \in 1...N}$ as input

    $\theta_t \leftarrow \theta_k$

    **for** $t$ in $0...N_{BATCH}$:

        $\hat{p}_i := \hat{P}[i], \hat{y}_{ij} := f_{\theta_t}(x_{ij}), y_i := h\left( \{y_{ij}\}_{j \in 1...m} \right)$

        $\mathcal{L}(\theta_t) = -\frac{1}{N_{Pos}} \sum_{i \in Pos}^{N_{Pos}} (\mathcal{C}_k(\hat{p}_i) \cdot w \cdot \log(\hat{y}_i)) - \frac{1}{N_{Neg}} \sum_{i \in Neg}^{N_{Neg}} \mathbb{E}_{j \sim P_i(X_i)} \log(1 - \hat{y}_{ij})$

        $\theta_t \leftarrow \nabla_{\theta_t} \mathcal{L}(\theta_t)$

---

    **end for**

    $\theta_k \leftarrow \theta_t$

**end for**

**return** $\theta$

---

It is important to note that, compared to prior art, the loss function $L(\theta)$ according to the invention explicitly accounts for the model confidence in the model predictions during training. In accordance with embodiments of the invention this is achieved by accounting for $\hat{p}_i$, the predicted probability of $\widehat{y_i}$. Specifically, in existing approaches, the loss can be attributed to wrong instances $x_{ij}$, therefore $f_\theta$ can be optimized to predict a "correct" label of the bag by predicting on the wrong instance $x_{ij}$.

Further, embodiments of the invention also extend prior art by including the function $\mathcal{C}$ for calibrating the predicted probabilities. The probabilities $\hat{p}_i$ can be calibrated by performing isotonic regression from the predicted logits (i.e. the logarithms of the odds ($\hat{p}_i/(1-\hat{p}_i)$)) and the labels on the training set. For instance, the isotonic regression may be performed according to the approach described in Barlow, R.E., 1972. Statistical inference under order restrictions; the theory and application of isotonic regression (No. 04; QA278. 7, B3.).

However, as will be appreciated by those skilled in the art, other approaches such as Platt's scaling could also be used. The applicability of the approach proposed in accordance with the present invention does not depend on the exact form of the calibration function.

The parameters $\theta$ of the model can then be learned using appropriate optimization techniques to minimize this loss function. For example, if $f_\theta$ is differentiable, such as with the BERT model, then gradient descent or similar algorithms can be used. According to an alternative embodiment, if $f_\theta$ is not differentiable, then Bayesian optimization or other black box methods can be used. The applicability of the approach proposed in accordance with the present invention does not depend on whether $f_\theta$ is differentiable.

After termination of the offline training phase as described above, an online prediction phase can be conducted. Specifically, after training, the model $f_\theta$ takes as input $X_i$ and predicts the label $y_i$. That is, the model takes as input a peptide sequence and a set of MHC molecules, and it predicts whether that peptide will be presented by any of those MHC molecules. According to embodiments it may be provided that the MIL classifier $f_\theta$ is used to make predictions for all combinations of peptide sequences and MHC molecules present in a biological sample. Based on thereupon, the peptides with the highest likelihood of being presented may be determined as candidates for being synthesized and included in a personalized cancer vaccine.

In practice, presentation of a peptide by an MHC molecule is only one (very important) step among many in ultimately creating an effective cancer vaccine. Predictive models for many of those steps do not obviously entail a multiple instance learning problem. Thus, the approach proposed in accordance with the embodiment of the present invention may be only applicable for parts of the vaccine design process. Furthermore, it should be noted that the proposed approach requires models which output some notion of probability. While this is common for classification problems, it is much less common for regression problems. Thus, the approach may be of limited use for multiple instance regression learning problems. Still further, it should be noted that most probability calibration functions require access to all uncalibrated probabilities. Thus, minibatch optimization approaches, which update the model after making predictions on only a few training samples, may not be compatible with certain embodiments of the present invention approach. Instead, embodiments of the invention train a calibration model at the beginning of each epoch.

The current state of the art for multiple instance learning for peptide-MHC presentation is the work by Reynisson, B., Alvarez, B., Paul, S., Peters, B. and Nielsen, M., 2020. NetMHCpan-4.1 and NetMHCIIpan-4.0: improved predictions of MHC antigen presentation by concurrent motif deconvolution and integration of MS MHC eluted ligand data. Nucleic Acids Research.

However, their approach does not incorporate the confidence weighting or calibration operations. Empirically, it could be demonstrated that the approach according to the present invention outperforms the approach by Reynisson et al. on a variety of datasets.

MHC class II binding data

In accordance with embodiments of the invention, to train the MHC class II binding model, the data from Jensen et al., 2018 (see Jensen, K. K., Andreatta, M., Marcatili, P., Buus, S., Greenbaum, J. A., Yan, Z., Sette, A., Peters, B., and Nielsen, M. (2018). Improved methods for predicting peptide binding affinity to MHC class II molecules. Immunology, 154(3), 394-406) were used, since it has been designed to minimize the overlap between the training and evaluation sets. The original data was collected from the Immune Epitope Database (IEDB, Vita, R., Mahajan, S., Overton, J. A., Dhanda, S. K., Martini, S., Cantrell, J. R., Wheeler, D. K., Sette, A., and Peters, B. (2019). The Immune Epitope Database (IEDB): 2018 update. Nucleic Acids Research, 47(D1), D339-D343, accessed on 30 June 2020) up to the year 2016. The data consists of 134 281 data points and covers HLA-DR, HLA-DQ, HLA-DP and H-2 mouse MHC allele. The affinity labels were transformed from IC50 to value between 0 and 1 with the fomula 1 - log(IC50)/ log(50 000).

The data from Jensen et al. was collected from IEDB up to the year 2016. To benchmark on an independent dataset where no model has been used for training or validation, quantitative binding data were collected from IEDB and data already used in Jensen et al. were filtered out. In addition, additional independent binding data from the Dana-Farber repository (for reference, see G. L., Lin, H. H., Keskin, D. B., Reinherz, E. L., and Brusic, V. (2011). Dana-farber repository for machine learning in immunology. Journal of immunological methods, 374(1-2), 28-25) were collected.

In the end, 2 413 additional MHC-peptide pairs covering 47 MHC class II alleles were collected.

MHC class II presentation data

To train a MHC class II mass spectrometry presentation model, the data curated from Reynisson, B., Alvarez, B., Paul, S., Peters, B., and Nielsen, M. (2020). NetMHCpan-4.1 and NetMHCIIpan-4.0: improved predictions of MHC antigen presentation by concurrent motif deconvolution and integration of MS MHC eluted ligand data. Nucleic Acids Research, pages 1-6, were used. The original data were curated from IEDB and other public sources. The data covers 41 MHC class II allele with peptide length ranging from 13 to 21. Each data point consists of the peptide ligand, the source protein and list of possible MHC class II allele bound to the peptide. The data points where only one MHC allele is unambiguously given are referred as single-allele data (SA), whereas the data points where multiple potential alleles, due to the nature of the mass spectrometry experiment, are given are referred as multi-allele data (MA). Reynisson et al., selected negative peptides by randomly sampling from the UniProt database. Peptide lengths for the negatives were sampled uniformly from 13 to 21.

According to embodiments of the invention, the MIL problem is tackled with an instance-level approach. Compared to a bag-level approach, this approach maximizes the model accuracy at predicting one single instance instead of a whole bag. Performance of an instance-level approach relies on correctly detecting the *key instance* (the positive instance in the positive bag). Therefore, a good instance-level model can not only be applied to the MIL problem but also to the single instance learning problem. In fact, in the peptide-MHC presentation problem, embodiments of the invention provide for using the same model jointly trained on single instance data and multiple instance data to maximize the usage of existing data. Previous work has shown that models which detect key instances also have better bag-level generalizability. Bag-level approaches, however, may have good performance at the bag-level, but are not guaranteed to generalize well to single instance cases. For biological applications, it is crucial for that the model is able to detect correctly the key instances.

In the following, some further example embodiments from several domains in which the invention can be used will be described.

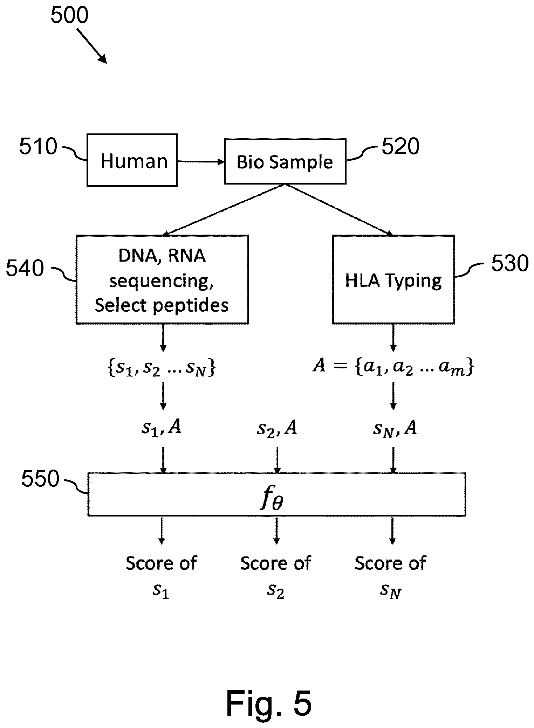

**Personalized cancer vaccine design.** This embodiment relates to a personalized cancer vaccine design system 500, which is schematically illustrated in Fig. 5, wherein the model is trained as described above. For prediction, the set of MHC molecules (generally denoted HLA, Human Leukocyte Antigen, Typing 530 in Fig. 5) is taken as the MHC molecules from a biological sample 520 taken from a specific patient 510, and the set of peptides 540 are based on mutations present in the cancerous cells of the patient 510. Predictions are made as described for all combinations of peptide and MHC pairs for that patient by using the trained MIL classifier $f_\theta$, as shown at 550. The peptides with the highest likelihood of being presented (i.e. with the highest scores, as indicated in Fig. 5) are then synthesized and included in a personalized cancer vaccine for that specific patient 510.

**Immune response prediction.** ELISpot is a widely-used immune response assay which measures if a particular peptide leads to an immune response when combined with a biological sample, such as blood from a patient infected with coronavirus. For example, interferon gamma is commonly measured with ELISpot. The immune response measurement from ELISpot is a result of interactions between the peptide and at least one of the MHC molecules present in the sample. According to an embodiment, the MIL approach discloses herein can also be used to train a model to predict this immune response. Compared to the formulation above, the only difference is that the bag labels are the results of the immune response assays. Such a model could also be used in a personalized cancer vaccine design system.

In further embodiments, a system for predicting binding and presentation of peptides by MHC molecules or a system for performing multiple instance learning comprises one or more processors which, alone or in combination, are configured to allow for execution of any of the methods according to embodiments of the present invention. In even further embodiments, a tangible, non-transitory computer-readable medium comprises instructions which, upon execution on one or more processors cause the one or more processors, alone or in combination, to allow for execution of any of the methods according to embodiments of the present invention. The processors can include one or more distinct processors, each having one or more cores, and access to memory. Each of the distinct processors can have the same or different structure. The processors can include one or more central processing units (CPUs), one or more graphics processing units (GPUs), circuitry (e.g., application specific integrated circuits (ASICs)), digital signal processors (DSPs), and the like. The processors can be mounted to a common substrate or to multiple different substrates. Processors are configured to perform a certain function, method, or operation (e.g., are configured to provide for performance of a function, method, or operation) at least when one of the one or more of the distinct processors is capable of performing operations embodying the function, method, or operation. Processors can perform operations embodying the function, method, or operation by, for example, executing code (e.g., interpreting scripts) stored on memory and/or trafficking data through one or more ASICs. Processors can be configured to perform, automatically, any and all functions, methods, and operations disclosed herein. Therefore, processors can be configured to implement any of (e.g., all) the protocols, devices, mechanisms, systems, and methods described herein. For example, when the present disclosure states that a method or device performs task "X" (or that task "X" is performed), such a statement should be understood to disclose that processor is configured to perform task "X".

Each of the computer entities can include memory. Memory can include volatile memory, non-volatile memory, and any other medium capable of storing data. Each of the volatile memory, non-volatile memory, and any other type of memory can include multiple different memory devices, located at multiple distinct locations and each having a different structure. Memory can include remotely hosted (e.g., cloud) storage. Examples of memory include a non-transitory computer-readable media such as RAM, ROM, flash memory, EEPROM, any kind of optical storage disk such as a DVD, magnetic storage, holographic storage, a HDD, a SSD, any medium that can be used to store program code in the form of instructions or data structures, and the like. Any and all of the methods, functions, and operations described in the present application can be fully embodied in the form of tangible and/or non-transitory machine-readable code (e.g., interpretable scripts) saved in memory.

The invention is set out in the appended claims.

**Claims**

1. A computer-implemented method for predicting binding and presentation of peptides by MHC molecules, the method comprising:

collecting or generating training data, wherein the training data includes a set of MHC molecules present in a biological sample as well as a set of observed peptide sequences that are presented by at least one of the MHC molecules present in the biological sample, wherein it is not known to which specific of the MHC molecules a peptide sequence is bound, wherein the training data is provided in form of a set of triples $\{s_i, A_i, y_i\}$, where $s_i$ is a peptide sequence, $A_i = \{a_1, \cdots, a_m\}$ is a set of m MHC molecules associated with a biological sample, and $y_i$ is a binary label indicating whether $s_i$ was found to be presented by any of the MHC molecules in $A_i$, and wherein the training data are organized in bags with each bag having a set of training instances, wherein labels are known for the bags, but unknown for the training instances;
using a loss function to train an MIL classifier $f_\theta$ at an instance-level, wherein the MIL classifier $f_\theta$ is trained to predict whether a particular peptide sequence will be presented by any of the MHC molecules present in the biological sample;
wherein the parameters of the MIL classifier $f_\theta$ are trained by a loss function $L(\theta)$ that includes a probability calibration function C to calibrate model confidence, the probability calibration function C being configured to predict in each training epoch $k + 1$ the probabilities $\hat{p}_i$ of $\hat{y}_i$ of the previous training epoch $k$, wherein

$$\hat{y}_i = \max_j(f_\theta(x_{ij}))$$ and $x_{ij}$ corresponds to the tuple $(s_i, a_i)$, where $s_i$ is the peptide and $a_i$ is the $j_{th}$ MHC molecule in $A_i$, wherein only individual training instances from positively labeled bags are weighted by the probability calibration function $\mathcal{C}$; and
predicting the label of new instances by applying the MIL classifier $f_\theta$ directly and/or predicting the label of new bags by applying the MIL classifier $f_\theta$ to each instance of a respective bag and aggregating the results among all instances of the respective bag.

2. The method according to claim 1, further comprising obtaining the training data from mass spectrometry experiments.

3. The method according to claim 1 or 2, further comprising:

providing, in a prediction phase after training, the MIL classifier $f_\theta$ a peptide sequence $s_i$ and a set of MHC molecules $a_i$ as input, and
predicting, by applying the MIL classifier $f_\theta$ to the input, whether that peptide sequence $s_i$ will be presented by any of those MHC molecules.

4. The method according to any of claims 1 to 3, further comprising:

using the MIL classifier $f_\theta$ to make predictions for all combinations of peptide sequences and MHC molecules present in the biological sample; and
determining the peptides with the highest likelihood of being presented as candidates for being synthesized and included in a personalized cancer vaccine.

5. A tangible, non-transitory computer-readable medium comprising instructions which, upon execution on one or more processors cause the one or more processors, alone or in combination, to allow for execution of the method according to any of claims 1 to 4.

6. A system for predicting binding and presentation of peptides by MHC molecules, the system comprising one or more processors which, alone or in combination, are configured to allow for execution of a method comprising:

collecting or generating training data, wherein the training data includes a set of MHC molecules present in a biological sample as well as a set of observed peptide sequences that are presented by at least one of the MHC molecules present in the biological sample, wherein it is not known to which specific of the MHC molecules a peptide sequence is bound, wherein the training data is provided in form of a set of triples $\{s_i, A_i, y_i\}$, where $s_i$ is a

peptide sequence, $A_i = \{a_1, \cdots, a_m\}$ is a set of $m$ MHC molecules associated with a biological sample, and $y_i$ is a binary label indicating whether $s_i$ was found to be presented by any of the MHC molecules in $A_i$;

organizing the training data in bags, with each bag having a set of training instances, wherein labels are known for the bags, but unknown for the training instances;

using a loss function to train an MIL classifier $f_\theta$ at an instance-level, wherein the MIL classifier $f_\theta$ is trained to predict whether a particular peptide sequence will be presented by any of the MHC molecules present in the biological sample;

training the parameters of the MIL classifier $f_\theta$ by a loss function $L(\theta)$ that includes a probability calibration function $\mathcal{C}$ to calibrate model confidence, the probability calibration function $\mathcal{C}$ being configured to predict in each training epoch $k + 1$ the probabilities $\hat{p}_i$ of $\hat{y}_i$ of the previous training epoch $k$, wherein $\hat{y}_i = \max_j(f_\theta(x_{ij}))$ and $x_{ij}$ corresponds to the tuple $(s_i, a_i)$, where $s_i$ is the peptide and $a_i$ is the $j_{th}$ MHC molecule in $A_i$, wherein only individual training instances from positively labeled bags are weighted by the probability calibration function $\mathcal{C}$; and

predicting the label of new instances by applying the MIL classifier $f_\theta$ directly and/or predicting the label of new bags by applying the MIL classifier $f_\theta$ to each instance of a respective bag and aggregating the results among all instances of the respective bag.

**Patentansprüche**

1. Computerimplementiertes Verfahren zur Vorhersage der Bindung und Präsentation von Peptiden durch MHC-Moleküle, wobei das Verfahren umfasst:

   Sammeln oder Erzeugen von Trainingsdaten, wobei die Trainingsdaten eine Menge von in einer biologischen Probe vorhandenen MHC-Molekülen sowie eine Menge von beobachteten Peptidsequenzen umfassen, die von mindestens einem der in der biologischen Probe vorhandenen MHC-Moleküle präsentiert werden, wobei nicht bekannt ist, an welches spezifische der MHC-Moleküle eine Peptidsequenz gebunden ist, wobei die Trainingsdaten in Form einer Menge von Tripeln $\{s_i, A_i, y_i\}$ bereitgestellt werden, wobei $s_i$ eine Peptidsequenz ist, $A_i = \{a_1, \cdots, a_m\}$ eine Menge von m mit einer biologischen Probe assoziierten MHC-Molekülen ist, und $y_i$ ein binäres Label ist, das angibt, ob $s_i$ von einem der MHC-Moleküle in $A_i$ präsentiert wurde, und wobei die Trainingsdaten in Bags organisiert sind, wobei jede Bag eine Menge von Trainingsinstanzen aufweist, wobei Labels für die Bags bekannt sind, jedoch für die Trainingsinstanzen unbekannt sind;

   Verwenden einer Verlustfunktion, um einen MIL-Klassifikator $f_\theta$ auf Instanzebene zu trainieren, wobei der MIL-Klassifikator $f_\theta$ trainiert wird, um vorherzusagen, ob eine bestimmte Peptidsequenz von einem der in der biologischen Probe vorhandenen MHC-Moleküle präsentiert wird;

   wobei die Parameter des MIL-Klassifikators $f_\theta$ durch eine Verlustfunktion $L(\theta)$ trainiert werden, die eine Wahr-scheinlichkeitskalibrierungsfunktion $\mathcal{C}$ zum Kalibrieren der Modellkonfidenz umfasst, wobei die Wahrscheinlichkeitskalibrierungsfunktion $\mathcal{C}$ so konfiguriert ist, dass sie in jeder Trainingsperiode $k + 1$ die Wahrscheinlichkeiten $\hat{p}_i$ von $\hat{y}_i$ der vorherigen Trainingsperiode $k$ vorhersagt, wobei $\hat{y}_i = \max_j(f_\theta(x_{ij}))$ und $x_{ij}$ dem Tupel $(s_i, a_i)$ entspricht, wobei $s_i$ das Peptid und $a_i$ das $j$-te MHC-Molekül in $A_i$ ist, wobei nur einzelne Trainingsinstanzen aus positiv gekennzeichneten Bags durch die Wahrscheinlichkeitskalibrierungsfunktion $\mathcal{C}$ gewichtet werden; und

   Vorhersagen des Labels neuer Instanzen durch direktes Anwenden des MIL-Klassifikators $f_\theta$ und/oder Vorher-sagen des Labels neuer Bags durch Anwenden des MIL-Klassifikators $f_\theta$ auf jede Instanz einer jeweiligen Bag und Aggregieren der Ergebnisse aller Instanzen der jeweiligen Bag.

2. Verfahren nach Anspruch 1, ferner umfassend das Erhalten der Trainingsdaten aus Massenspektrometrie-Experimenten.

3. Verfahren nach Anspruch 1 oder 2, das ferner umfasst:

   Bereitstellen einer Peptidsequenz $s_i$ und einer Menge von MHC-Molekülen $a_i$ als Eingabe für den MIL-Klassifika-tor $f_\theta$ in einer Vorhersagephase nach dem Training, und
   Vorhersagen, durch Anwenden des MIL-Klassifikators $f_\theta$ auf die Eingabe, ob die Peptidsequenz $s_i$ durch eines

der MHC-Moleküle präsentiert wird.

4. Verfahren nach einem der Ansprüche 1 bis 3, das ferner umfasst:

Verwenden des MIL-Klassifikators $f_\theta$, um Vorhersagen für alle Kombinationen von Peptidsequenzen und in der biologischen Probe vorhandenen MHC-Molekülen zu treffen; und
Bestimmen der Peptide mit der höchsten Wahrscheinlichkeit, präsentiert zu werden, als Kandidaten zur Synthese und Aufnahme in einen personalisierten Krebsimpfstoff.

5. Greifbares, nicht flüchtiges, computerlesbares Medium, das Anweisungen enthält, die bei Ausführung auf einem oder mehreren Prozessoren bewirken, dass der eine oder die mehreren Prozessoren allein oder in Kombination die Ausführung des Verfahrens gemäß einem der Ansprüche 1 bis 4 ermöglichen.

6. System zur Vorhersage der Bindung und Präsentation von Peptiden durch MHC-Moleküle, wobei das System einen oder mehrere Prozessoren umfasst, die allein oder in Kombination so konfiguriert sind, dass sie die Ausführung eines Verfahrens ermöglichen, das Folgendes umfasst:

Sammeln oder Erzeugen von Trainingsdaten, wobei die Trainingsdaten eine Menge von in einer biologischen Probe vorhandenen MHC-Molekülen sowie eine Menge von beobachteten Peptidsequenzen umfassen, die von mindestens einem der in der biologischen Probe vorhandenen MHC-Moleküle präsentiert werden, wobei nicht bekannt ist, an welches spezifische der MHC-Moleküle eine Peptidsequenz gebunden ist, wobei die Trainingsdaten in Form einer Menge von Tripeln $\{s_i, A_i, y_i\}$ bereitgestellt werden, wobei $s_i$ eine Peptidsequenz ist, $A_i = \{a_1, \cdots, a_m\}$ eine Menge von m mit einer biologischen Probe assoziierten MHC-Molekülen ist, und $y_i$ ein binäres Label ist, das angibt, ob $s_i$ von einem der MHC-Moleküle in $A_i$ präsentiert wurde;
Organisieren der Trainingsdaten in Bags, wobei jede Bag eine Menge von Trainingsinstanzen aufweist, wobei Labels für die Bags bekannt sind, jedoch für die Trainingsinstanzen unbekannt sind;
Verwenden einer Verlustfunktion, um einen MIL-Klassifikator $f_\theta$ auf Instanzebene zu trainieren, wobei der MIL-Klassifikator $f_\theta$ trainiert wird, um vorherzusagen, ob eine bestimmte Peptidsequenz von einem der in der biologischen Probe vorhandenen MHC-Moleküle präsentiert wird;
Trainieren der Parameter des MIL-Klassifikators $f_\theta$ durch eine Verlustfunktion $L(\theta)$, die eine Wahrscheinlichkeitskalibrierungsfunktion $\mathcal{C}$ zum Kalibrieren der Modellkonfidenz umfasst, wobei die Wahrscheinlichkeitskalibrierungsfunktion $\mathcal{C}$ so konfiguriert ist, dass sie in jeder Trainingsperiode $k + 1$ die Wahrscheinlichkeiten $\hat{p}_i$ von $\hat{y}_i$ der vorherigen Trainingsperiode $k$ vorhersagt, wobei $\hat{y}_i = \max_j(f_\theta(x_{ij}))$ und $x_{ij}$ dem Tupel $(s_i, a_i)$ entspricht, wobei $s_i$ das Peptid und $a_i$ das $j$-te MHC-Molekül in $A_i$ ist, wobei nur einzelne Trainingsinstanzen aus positiv gekennzeichneten Bags durch die Wahrscheinlichkeitskalibrierungsfunktion $\mathcal{C}$ gewichtet werden; und
Vorhersagen des Labels neuer Instanzen durch direktes Anwenden des MIL-Klassifikators $f_\theta$ und/oder Vorhersagen des Labels neuer Bags durch Anwenden des MIL-Klassifikators $f_\theta$ auf jede Instanz einer jeweiligen Bag und Aggregieren der Ergebnisse aller Instanzen der jeweiligen Bag.

**Revendications**

1. Procédé mis en œuvre par ordinateur pour prédire la liaison et la présentation de peptides par des molécules MHC, le procédé comportant :

la collecte ou la génération de données d'entraînement, dans lequel les données d'entraînement incluent un ensemble de molécules MHC présentes dans un échantillon biologique ainsi qu'un ensemble de séquences peptidiques observées qui sont présentées par au moins l'une des molécules MHC présentes dans l'échantillon biologique, dans lequel on ne sait pas à laquelle des molécules MHC spécifique une séquence peptidique est liée, dans lequel les données d'entraînement sont fournies sous la forme d'un ensemble de triplets $\{s_i, A_i, y_i\}$, où $s_i$ est une séquence peptidique, $A_i = \{a_1, ..., a_m\}$ est un ensemble de m molécules MHC associées à un échantillon biologique, et $y_i$ est une étiquette binaire indiquant s'il a été constaté que $s_i$ était présentée par l'une quelconque des molécules MHC dans $A_i$, et dans lequel les données d'entraînement sont organisées en sacs avec chaque sac comportant un ensemble d'instances d'entraînement, dans lequel les étiquettes sont connues pour les sacs, mais inconnues pour les instances d'entraînement ;
l'utilisation d'une fonction de perte pour entraîner un classificateur MIL $f_\theta$ à un niveau d'instance, dans lequel le

classificateur MIL $f_\theta$ est entraîné pour prédire si une séquence peptidique particulière sera présentée par l'une quelconque des molécules MHC présentes dans l'échantillon biologique ;

dans lequel les paramètres du classificateur MIL $f_\theta$ sont entraînés par une fonction de perte $L(\theta)$ qui inclut une fonction d'étalonnage de probabilité $\mathcal{C}$ pour étalonner une confiance de modèle, la fonction d'étalonnage de probabilité $\mathcal{C}$ étant configurée pour prédire, à chaque époque d'entraînement $k + 1$, les probabilités $\hat{p}_i$ de $\hat{y}_i$ de l'époque d'entraînement précédente $k$, dans lequel $\hat{y}_i = \max_j \left( f_\theta(X_{ij}) \right)$ et $X_{ij}$ correspond au tuple $(s_i, a_i)$, où $s_i$ est le peptide et $a_i$ est la $j_{ème}$ molécule MHC dans $A_i$, dans lequel seules les instances d'entraînement individuelles de sacs étiquetés positivement sont pondérées par la fonction d'étalonnage de probabilité C ; et la prédiction de l'étiquette de nouvelles instances en appliquant directement le classificateur MIL $f_\theta$ et/ou la prédiction de l'étiquette de nouveaux sacs en appliquant le classificateur MIL $f_\theta$ à chaque instance d'un sac respectif et en agrégeant les résultats parmi toutes les instances du sac respectif.

2. Procédé selon la revendication 1, comportant en outre l'obtention des données d'entraînement à partir d'expériences de spectrométrie de masse.

3. Procédé selon la revendication 1 ou 2, comportant en outre :

la fourniture, dans une phase de prédiction après entraînement, du classificateur MIL $f_\theta$ d'une séquence peptidique $s_i$ et d'un ensemble de molécules MHC $a_i$ en tant qu'entrée, et
la prédiction, en appliquant le classificateur MIL $f_\theta$ à l'entrée, du fait que cette séquence peptidique $s_i$ sera présentée ou non par l'une quelconque de ces molécules MHC.

4. Procédé selon l'une quelconque des revendications 1 à 3, comportant en outre :

l'utilisation du classificateur MIL $f_\theta$ pour réaliser des prédictions pour toutes les combinaisons de séquences peptidiques et de molécules MHC présentes dans l'échantillon biologique ; et
la détermination des peptides présentant la probabilité la plus élevée d'être présentés comme candidats pour être synthétisés et inclus dans un vaccin contre le cancer personnalisé.

5. Support tangible, non transitoire, lisible par ordinateur, comportant des instructions qui, lorsqu'elles sont exécutées sur un ou plusieurs processeurs, amènent les un ou plusieurs processeurs, seuls ou en combinaison, à permettre l'exécution du procédé selon l'une quelconque des revendications 1 à 4.

6. Système de prédiction de la liaison et de la présentation de peptides par des molécules MHC, le système comportant un ou plusieurs processeurs qui, seuls ou en combinaison, sont configurés pour permettre l'exécution d'un procédé comportant :

la collecte ou la génération de données d'entraînement, dans lequel les données d'entraînement incluent un ensemble de molécules MHC présentes dans un échantillon biologique ainsi qu'un ensemble de séquences peptidiques observées qui sont présentées par au moins l'une des molécules MHC présentes dans l'échantillon biologique, dans lequel on ne sait pas à laquelle des molécules MHC spécifique une séquence peptidique est liée, dans lequel les données d'entraînement sont fournies sous la forme d'un ensemble de triplets $\{s_i, A_i, y_i\}$, où $s_i$ est une séquence peptidique, $A_i = \{a_1, ..., a_m\}$ est un ensemble de $m$ *molécules* MHC associées à un échantillon biologique, et $y_i$ est une étiquette binaire indiquant s'il a été constaté que $s_i$ a été présentée par l'une quelconque des molécules MHC dans $A_i$ ;
l'organisation des données d'entraînement en sacs, chaque sac comportant un ensemble d'instances d'entraînement, dans lequel des étiquettes sont connues pour les sacs, mais inconnues pour les instances d'entraînement ;
l'utilisation d'une fonction de perte pour entraîner un classificateur MIL $f_\theta$ à un niveau d'instance, dans lequel le classificateur MIL $f_\theta$ est entraîné pour prédire si une séquence peptidique particulière sera présentée par l'une quelconque des molécules MHC présentes dans l'échantillon biologique ;
l'entraînement des paramètres du classificateur MIL $f_\theta$ par une fonction de perte $L(\theta)$ qui comporte une fonction d'étalonnage de probabilité $\mathcal{C}$ pour étalonner une confiance de modèle, la fonction d'étalonnage de probabilité $\mathcal{C}$ étant configurée pour prédire à chaque époque d'entraînement $k + 1$ les probabilités $\hat{p}_i$ de $\hat{y}_i$ de l'époque

d'entraînement précédente $k$, dans lequel $\hat{y}_i = \max_j \left( f_\theta \left( X_{ij} \right) \right)$ et $x_{ij}$ correspondent au tuple $(s_i, a_i)$, où $s_i$ est le peptide et $a_i$ est la $j_{ème}$ molécule MHC dans $A_i$, dans lequel seules les instances d'entraînement individuelles de sacs étiquetés positivement sont pondérées par la fonction d'étalonnage de probabilité $\mathcal{C}$ ; et

la prédiction de l'étiquette de nouvelles instances en appliquant directement le classificateur MIL $f_\theta$ et/ou la prédiction de l'étiquette de nouveaux sacs en appliquant le classificateur MIL $f_\theta$ à chaque instance d'un sac respectif et en agrégeant les résultats parmi toutes les instances du sac respectif.

# Fig. 1

BAG $X_i$ $\boxed{s_i}$ $\boxed{a_{i1}}$ $\cdots$ $\boxed{s_i}$ $\boxed{a_{im}}$

$f_{\theta_t}$ $\cdots$ $f_{\theta_t}$

$$\hat{y}_{ij} := f_{\theta_t}(x_{i1}) \qquad f_{\theta_t}(x_{im})$$

$$h$$

$$\hat{y}_i := f_{\theta_t}(x_i)$$

# Fig. 2

$$\hat{y}_1^k \qquad \hat{y}_2^k \qquad \cdots \qquad \hat{y}_N^k$$

$$\mathcal{C}$$

$$\mathcal{C}(\hat{p}_1^{k+1}) \qquad \mathcal{C}(\hat{p}_2^{k+1}) \qquad \mathcal{C}(\hat{p}_N^{k+1})$$

# Fig. 3

$$X_{Pos} \qquad\qquad\qquad X_{Neg}$$

$$\{x_{i1} \dots x_{im}\}, y_i = 1 \qquad\qquad \{x_{i1} \dots x_{im}\}, y_i = 0$$

$$y_{ij} = 0$$

Confidence Weighting
$$\mathcal{C}(\hat{p}_i)$$

$$\mathcal{L}(\theta)$$
$$= -\frac{1}{N_{Pos}} \sum_{i \in Pos}^{N_{Pos}} \left( \mathcal{C}(\hat{p}_i) \cdot w \cdot \log(\hat{y}_i) \right) - \frac{1}{N_{Neg}} \sum_{i \in Neg}^{N_{Neg}} \mathbb{E}_{j \sim P_i(X_i)} \log(1 - \hat{y}_{ij})$$

# Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150278441 A1

**Non-patent literature cited in the description**

- **MURPHY, K.** ; **WEAVER, C.** Janeway's immunobiology. Garland science, 2016
- **CHOO, S.Y.** The HLA system: genetics, immunology, clinical testing, and clinical implications. *Yonsei medical journal*, 2007, vol. 48 (1), 11-23
- **PURCELL, A.W.** ; **RAMARATHINAM, S.H.** ; **TERNETTE, N.** Mass spectrometry-based identification of MHC-bound peptides for immunopeptidomics. *Nature protocols*, 2019, vol. 14 (6), 1687
- **VITA, R.** ; **MAHAJAN, S.** ; **OVERTON, J.A.** ; **DHANDA, S.K.** ; **MARTINI, S.** ; **CANTRELL, J.R.** ; **WHEELER, D.K.** ; **SETTE, A.** ; **PETERS, B.** The immune epitope database (IEDB): 2018 update. *Nucleic acids research*, 2019, vol. 47 (D1), D339-D343
- **YASSER EL-MANZALAWY et al.** Predicting MHC-II Binding Affinity Using Multiple Instance Regression. *IEEE/ACM Transactions on Computational Biology and Bioinformatics*, August 2011, vol. 8 (4), 1067-1079
- Multiple Instance Learning Allows MHC Class II Epitope Predictions Across Alleles. **NICO PFEIFER** ; **OLIVER KOHLBACHER.** Algorithms in Computer Science. Springer-Verlag Berlin Heidelberg, 15 September 2008, 210-221
- **YICHANG XU et al.** MHC2MIL: a novel multiple instance learning based method for MHC-II peptide binding prediction by considering peptide flanking region and residue positions. *BMC Genomics*, 08 December 2014, vol. 15
- **PETERS, M. E.** ; **NEUMANN, M.** ; **LYYER, M.** ; **GARDNER, M.** ; **CLARK, C.** ; **LEE, K.** ; **ZETTLEMOYER, L.** Deep contextualized word representations. *Proceedings of the 16th Annual Conference of the North American Chapter of the Association for Computational Linguistics: Human Language Technologies*, 2018, vol. 1, 2227-2237

- **BARLOW, R.E.** *Statistical inference under order restrictions; the theory and application of isotonic regression*, 1972
- **REYNISSON, B.** ; **ALVAREZ, B.** ; **PAUL, S.** ; **PETERS, B.** ; **NIELSEN, M.** NetMHCpan-4.1 and NetMHCIIpan-4.0: improved predictions of MHC antigen presentation by concurrent motif deconvolution and integration of MS MHC eluted ligand data. *Nucleic Acids Research*, 2020
- **JENSEN, K. K.** ; **ANDREATTA, M.** ; **MARCATILI, P.** ; **BUUS, S.** ; **GREENBAUM, J. A.** ; **YAN, Z.** ; **SETTE, A.** ; **PETERS, B.** ; **NIELSEN, M.** Improved methods for predicting peptide binding affinity to MHC class II molecules. *Immunology*, 2018, vol. 154 (3), 394-406
- **VITA, R.** ; **MAHAJAN, S.** ; **OVERTON, J. A.** ; **DHANDA, S. K.** ; **MARTINI, S.** ; **CANTRELL, J. R.** ; **WHEELER, D. K.** ; **SETTE, A.** ; **PETERS, B.** The Immune Epitope Database (IEDB): 2018 update. *Nucleic Acids Research*, 2019, vol. 47 (D1), D339-D343
- **G. L.** ; **LIN, H. H.** ; **KESKIN, D. B.** ; **REINHERZ, E. L.** ; **BRUSIC, V.** Dana-farber repository for machine learning in immunology. *Journal of immunological methods*, 2011, vol. 374 (1-2), 28-25
- **REYNISSON, B.** ; **ALVAREZ, B.** ; **PAUL, S.** ; **PETERS, B.** ; **NIELSEN, M.** NetMHCpan-4.1 and NetMHCIIpan-4.0: improved predictions of MHC antigen presentation by concurrent motif deconvolution and integration of MS MHC eluted ligand data. *Nucleic Acids Research*, 2020, 1-6